# FASCICULE DE BREVET EUROPEEN

(11) **EP 0 968 702 B1**
(45) Date de publication et mention de la délivrance du brevet: **06.12.2000**
(21) Numéro de dépôt: 99401135.1
(22) Date de dépôt: 07.05.1999
(51) Int. Cl.: A61K 7/00, A61K 7/50

(54) **Composition pulvérulente moussante solide à hydratrer pour le soin ou le nettoyage de la peau**
Pulverförmige feste schaumbildende Befeuchtungszusammensetzung für die Pflege oder Reinigung der Haut
Foaming solid powdery composition to be hydrated for use in skin-care or skin-cleansing

(30) Priorité: 15.06.1998 FR 9807519
(43) Date de publication de la demande: 05.01.2000
(73) Titulaire: L'OREAL, 75008 Paris (FR)
(72) Inventeur: Roulier, Véronique, 75010 Paris (FR); Daubige, Thérèse, 77480 Mousseaux les Bray (FR); Guillou, Véronique, 92600 Asnieres (FR)
(74) Mandataire: Rasson, Catherine

(56) Documents cités:
- EP-A- 0 745 379
- DE-A- 4 420 880

## Description

La présente invention a pour objet une composition pulvérulente moussante pour le nettoyage en profondeur et/ou le soin de la peau ayant un aspect de solide déformable. Cette composition peut être appliquée aussi bien sur le visage que sur le corps humain et est d'une très grande douceur. Cette composition de toucher sec doit être hydratée avant usage.

L'invention se rapporte aussi à un procédé cosmétique de nettoyage et/ ou de soin de la peau, ainsi qu'à un procédé de fabrication de la composition pulvérulente.

Les compositions nettoyantes pour la peau se présentent habituellement sous forme de pains solides comme les savons ou sous forme de liquides plus ou moins visqueux.

Pour se nettoyer avec un savon, les consommateurs se servent en général du savon entier. Ce savon a tendance à se ramollir au fur et à mesure de ses utilisations, du fait de son contact avec l'eau, et à mal vieillir. En outre, il est fréquent qu'il se casse et que les consommateurs se retrouvent avec des petits bouts de savon difficiles à utiliser. Par ailleurs, un savon mouillé est généralement glissant, ce qui rend son emploi malaisé, notamment pour les jeunes enfants. De ce fait, il est devenu courant d'utiliser des nettoyants liquides à la place de savons. Malheureusement, plus les compositions sont liquides, plus il est difficile de les doser, notamment parce qu'elles ont tendance à s'échapper entre les doigts, et plus elles ont tendance à s'échapper de leur conditionnement, ce qui peut être très gênant lorsqu'elles viennent au contact de vêtement, lors de déplacement.

Il est aussi connu d'utiliser des masques nettoyants pour le nettoyage en profondeur du visage. Ces masques se présentent généralement sous forme de gel ou crème à appliquer en couche mince sur la peau, contenant des poudres capables d'absorber les corps gras produits par la peau comme le sébum. Ces masques peuvent éventuellement contenir des actifs cosmétiques ou dermatologiques de la peau pour parfaire le nettoyage et/ou apporter du bien être à la peau. De tels masques sont notamment décrits dans les documents US-A-5,690,945 et WO-A-86/05394. Ces masques sont souvent lourds à porter, peu confortables (tiraillement) et le nettoyage n'est pas toujours ressenti comme efficace. De plus, ces masques sont difficilement rinçables.

Par ailleurs, les utilisateurs recherchent, de plus en plus, de nouvelles textures et de nouveaux type de produits.

La présente invention a justement pour objet une nouvelle composition pour le nettoyage et/ou le soin de la peau permettant notamment de remédier aux inconvénients mentionnés ci-dessus. Cette composition est une composition moussante, d'application aisée, d'une très grande légèreté et d'une grande douceur, donnant un effet de bien être après application. Elle a, en outre, l'avantage de se rincer de façon remarquable et de présenter une texture tout à fait inhabituelle.

Ainsi, l'invention se rapporte à une composition pulvérulente moussante à hydrater pour le nettoyage et/ou le soin de la peau, contenant un liant cosmétiquement acceptable, et une charge pulvérulente insoluble dans ce liant et en quantité suffisante pour structurer ladite composition et lui conférer un aspect de solide déformable dans lequel le liant est emprisonné, la charge pulvérulente comprenant des particules solides de polymère thermoplastique et au moins un tensioactif sous forme pulvérulente, la dite composition étant apte à mousser lors de son hydratation.

Cette composition est destinée plus spécialement au nettoyage et/ou soin du visage d'être humain. Elle se présente sous forme de solide déformable ou malléable, sec, ne tachant pas et ressemblant à de la guimauve (voir le document US-A-3,682,659 pour la consistance de la guimauve). Ce solide peut être modelé comme de la pâte à modeler pour enfants. Il peut être rompu facilement à la main afin de ne prélever que la quantité nécessaire de produit. En particulier, cette composition peut être conditionnée sous forme de monodose, particulièrement avantageuse d'un point de vue hygiénique, et par exemple sous forme de petits cubes, de billes ou de berlingots.

Grâce à cette texture solide, la composition de l'invention ne risque pas de s'échapper de son conditionnement notamment lors de son transport. Par ailleurs cette composition est très facilement préhensible et ne s'écoule pas entre les doigts ; son dosage est beaucoup plus simple que celui des nettoyants liquides habituels et le problème d'usure des savons durs ne se pose pas. De plus, son application est aisée, légère et agréable. Par ailleurs, son stockage ne pose pas de problème et sa pollution par l'environnement et/ou la manipulation par le consommateur est relativement réduite et en tout état de cause très inférieure à celles des produits comparables de l'art antérieur. En particulier, il n'est pas nécessaire d'introduire de conservateur pour assurer sa protection antimicrobienne.

Grâce aux particules de l'invention, il est notamment possible d'obtenir une structure homogène (solide déformable) pour des constituants conduisant normalement à deux phases distinctes (constituants non miscibles par exemple huile/eau).

L'agent texturant de l'invention présente la particularité de s'éliminer facilement de la peau par simple dilution. Il joue en fait le rôle de véhicule ou de réservoir pour le liant cosmétique. Il permet, en outre, de récupérer le liant et notamment le ou les actifs, emprisonnés dans le solide déformable, lorsque c'est nécessaire par simple dilution à l'eau. Ceci est probablement dû au fait que le support cosmétique est logé dans les espaces interparticulaires du solide et non dans les particules.

En vue d'obtenir un solide au toucher agréable et doux, il est préférable d'utiliser des particules ayant une granulométrie de 1 µm à 300 µm, par exemple de 5 µm à 200 µm et de préférence de 10 µm à 100 µm et mieux de 15 µm à 40 µm.

La grande douceur apportée par ces particules permet l'utilisation de la composition de l'invention par des personnes à peau sensible.

Grâce à la présence du tensioactif et du polymère thermoplastique sous forme pulvérulente, le nettoyage se fait en profondeur et tout en douceur, sans dessécher et tirailler la peau.

Afin de conférer au masque de l'invention, un aspect aéré et léger, on utilise avantageusement, des particules ayant une densité inférieure à 0,09 et mieux inférieure à 0,06 et encore mieux inférieure à 0,04.

En vue d'obtenir cette faible densité, on utilise avantageusement des particules creuses remplies d'un gaz. Ce gaz peut être de l'air, de l'azote, de l'isobutane, de l'isopentane, etc.

Selon une autre caractéristique avantageuse de l'invention, les particules se présentent sous forme de billes. Il est toutefois possible d'utiliser des particules ayant la forme de fibres.

Ces particules peuvent être réalisées en différents matériaux inertes ne réagissant pas chimiquement avec le liant cosmétiquement acceptable ; en particulier ces particules ne réagissent pas avec les huiles, les tensioactifs, l'eau et les différents autres constituants de la composition, comme les actifs.

La poudre de l'invention, conférant la texture de solide déformable, a la particularité de se déliter facilement par simple dilution dans un solvant comme l'eau éventuellement chargée en sels ou oligo-éléments.

Comme critère de choix de la poudre, on peut réaliser le test suivant :
- ajout de particules déterminées dans de l'eau contenant un colorant classiquement utilisé dans le domaine nettoyant tel que le sel disodique de bleu brillant FCF codifié dans le Color Index sous la référence Cl 42090, jusqu'à l'obtention d'une pâte colorée,
- versement d'une goutte d'eau sur la pâte.

Lorsque la pâte au point d'impact de la goutte d'eau est beaucoup plus claire que le reste de la pâte, cela signifie que les particules considérées sont appropriées pour texturer la composition. Inversement, lorsque la pâte au point d'impact ne s'est pas décolorée, les particules considérées ne sont nullement appropriées.

Les particules inertes sont avantageusement réalisées en matériaux thermoplastiques comme les polyamides tels que le Nylon, les polymères ou copolymères d'acrylonitrile, de chlorure de vinylidène, de chlorure de vinyle et/ou de monomère acrylique ou styrénique, éventuellement expansés. Le monomère acrylique est par exemple un acrylate ou méthacrylate de méthyle ou d'éthyle. Le monomère styrénique est par exemple l'α-méthyl-styrène ou le styrène.

Comme particules de Nylon, on peut utiliser les particules d"Orgasol" vendues par la société Atochem. Ces particules sont des sphères pleines, poreuses, de diamètre allant de 5 µm à 60 µm.

De préférence, les particules sont des particules creuses déformables de copolymère expansé de chlorure de vinylidène et d'acrylonitrile ou de chlorure de vinylidène, d'acrylonitrile et de méthacrylate. On peut par exemple utiliser un copolymère contenant de 0 % à 60 % de motifs dérivés du chlorure de vinylidène, de 20 % à 90% de motifs dérivés d'acrylonitrile et de 0 % à 50 % de motifs dérivés d'un monomère acrylique ou styrénique, la somme des pourcentages (en poids) étant égale à 100. Ces particules se présentent notamment à l'état sec ou hydraté et peuvent être obtenues, par exemple, selon les procédés décrits dans les brevets et demandes de brevet EP-A-56 219, EP-A-348 572, EP-A-320 473, EP-A-112 807 et US-A-3,615,972.

Ces particules creuses peuvent être par exemple celles formées d'un terpolymère de chlorure de vinylidène, d'acrylonitrile et de méthacrylate, vendues sous la marque Expancel par la société Nobel Casco sous les références 551 DE 12 (granulométrie d'environ 12 µm et masse volumique 40 kg/m³), 551 DE 20 (granulométrie d'environ 30 µm et masse volumique 65 kg/m³), 551 DE 50 (granulométrie d'environ 40 µm). On peut aussi citer les microsphères formées du même terpolymère expansé à l'état sec ayant une granulométrie d'environ 18 µm et une masse volumique d'environ 60 à 80 kg/m³, appelées ci-dessous EL 23, ou ayant une granulométrie d'environ 34 µm et une masse volumique d'environ 20 kg/m³, appelées ci-dessous EL 43, ou ayant une granulométrie d'environ 150 µm, appelées ci-dessous EL 55.

Comme autres particules creuses polymériques utilisables dans l'invention, on peut encore citer les polymères et les copolymères obtenus à partir des esters ou acides, itaconique, citraconique, maléique, fumarique, de l'acétate ou lactate de vinyle (voir à cet effet le document JP-A-2-112304), ou encore des particules de copolymère non expansé de chlorure de vinylidène et d'acrylonitrile ou de chlorure de vinylidène, d'acrylonitrile et de méthacrylate, vendues sous la marque Expancel avec la référence 551 WU.

En revanche, les particules d'amidon de maïs, de silice pyrogénée, de polyéthylène, de polyuréthanne ou de polyester non expansé ne permettent pas d'obtenir une composition solide qui s'élimine bien de la peau lors du rinçage.

L'obtention ou non d'un solide déformable est liée à la quantité de poudre ou agent structurant utilisée dans le masque ; au-dessus d'une certaine quantité de particules, appelée charge pigmentaire volumique critique et notée CPVC on note une augmentation brusque de la viscosité du milieu. La CPVC est fonction du milieu et de la nature des particules ; elle doit donc être déterminée à chaque fois. Sa détermination ne pose aucun problème pour l'homme du métier. On peut, par exemple, utiliser la méthode officielle ASTM pour déterminer la CPVC.

En pratique la charge pulvérulente structurante représente jusqu'à 80 % en volume de la composition, dont avantageusement 60 % représente des particules ayant une densité inférieure à 1. Dans ces conditions, le liant représente jusqu'à 20 % en volume de la composition. En particulier, les particules de densité inférieure à 1 représentent de 2 à 20 % et mieux de 3 à 7 % du poids total de la composition.

L'invention a encore pour objet l'utilisation cosmétique de la composition définie précédemment pour le nettoyage et/ou le soin de la peau d'êtres humains.

Ainsi, l'invention a encore pour objet un procédé cosmétique de nettoyage et/ou de soin de la peau, consistant à hydrater une composition telle que définie ci-dessus, à la faire mousser, à l'appliquer sur la peau, et enfin à rincer la peau.

La composition de l'invention contient, outre, les particules de polymère, un tensioactif sous forme pulvérulente, en une quantité efficace lors de l'hydratation de la composition. Ce type d'agent présente la particularité de réagir chimiquement avec l'eau et l'agent structurant pour former une mousse facilement applicable sur la peau.

La composition selon l'invention peut avantageusement contenir un ou plusieurs tensioactifs nettoyants et/ou moussants sous forme pulvérulente, qui peuvent être des tensioactifs non ioniques, anioniques, cationiques et/ou amphotères. Ils peuvent être utilisés en une quantité allant par exemple de 10 % à 80 % du poids total de la composition, et de préférence de 30 % à 60 %.

De façon avantageuse, le ou les tensioactifs sous forme de poudre présentent un granulométrie allant de 5 à 50 µm et mieux de 10 à 20 µm.

La composition de l'invention peut avantageusement contenir, de plus, un ou plusieurs tensioactifs liquides pouvant représenter de 20 à 88 % du poids total de la composition et mieux de 33 à 67 %. Ces tensioactifs liquides font du partie du liant et peuvent même constituer la totalité du liant.

Comme tensioactifs non ioniques utilisables dans l'invention, on peut citer par exemple les condensats d'oxydes d'alkylène et d'alkyl phénols comme l'octyl phénol éthoxylé tel que celui vendu sous le nom Triton X45 par la société Rohm et Hass se présentant sous forme anhydre puvérulente, les condensats d'oxyde d'éthylène, d'oxyde de propylène et d'éthylène diamine, les alkylpolyglucosides, les éthers d'alcool gras et de polyols comme par exemple le Polyglyceryl-3 hydroxylauryl Ether (nom CTFA) vendu sous la dénomination Chimexane NF par la Société Chimex.

Comme tensioactifs anioniques, on peut citer par exemple les polyalkylènes glycols éther d'alcools, les taurates, les acyl lactylates comme le stéaroyl lactylate de sodium (par exemple Pationic SSL, tensioactif pulvérulent vendu par la société Maprecos), les alkyl sulfates comme le lauryl sulfate de sodium (Sipon LCS, tensioactif pulvérulent vendu par la société Henkel), les alkyl sulfates polyoxyéthylénés, les alkyl éthers sulfates comme le lauryl éther sulfate de monoéthanolamine notamment celui vendu à 28 % dans l'eau par la société Henkel (Sipon LEM 235), les alkyl éthers carboxylates, les monoalkyl ou dialkyl phosphates comme le monohexyl-2-décyl phosphate d'argine (C6C10 MAP-1-ARG, poudre vendue par la société Kao Chemicals), les alkyl phosphate éthoxylés, les N-acyl sarcosinates comme le lauroyl sarcosinate de sodium (par exemple Oramix L30 vendu par la société Seppic) ou le myristoyl sarcosinate de sodium (par exemple Nikkol Sarcosinate MN, poudre vendue par la société Nikko), les N-acyl glutamates comme le lauroyl glutamate de sodium (par exemple Amisoft LS11, poudre vendue par la société Ajinomoto), les acyl iséthionates comme le cocoyl iséthionate de sodium (vendu notamment par la société Rhône-Poulenc (Gerapon AC78) ou celui vendu en poudre par la société Jordan (Jordapon Cl)), les polysorbates, les succinamates, les savons comme le laurate, myristate, palmitate ou stéarate de potassium, leurs mélanges.

Comme tensioactifs amphotères ou zwitterions, on peut citer par exemple les bétaïnes et les dérivés de bétaïnes, les sultaïnes et les dérivés de sultaïnes, les dérivés imidazolinium, comme le disodium cocoamphodiacétate (Miranol C2M concentré vendu par la société Rhone-Poulenc).

Comme tensioactifs cationiques utilisables dans l'invention, on peut citer les dérivés de pyrrolidone carboxylate comme le PCA éthyl cocoyl arginate (Cation CAE, poudre vendue par la société Ajinomoto).

La composition selon l'invention peut, en outre, contenir des huiles qui peuvent être utilisées en une quantité allant de 0 % à 30 % en poids, et de préférence de 0 % à 10 % du poids total de la composition. Les huiles font partie intégrante du liant.

Les huiles utilisables dans les compositions selon rinvention peuvent être choisies parmi les huiles minérales comme l'huile de paraffine et l'huile de vaseline ; les huiles d'origine animale comme le perhydrosqualène ; les huiles d'origine végétale telles que l'huile d'amande douce, d'avocat, de ricin, d'olive, de jojoba, de sésame, d'arachide, de pépins de raisin, de colza, de coprah, de noisettes, de palme, de noyau d'abricot, de calophyllum, de son de riz, de germes de maïs, de germes de blé, de soja, de tournesol, de carthame, de passiflore, de seigle et le beurre de karité et sa fraction liquide ; les huiles de synthèse telles que les esters gras comme le myristate de butyle ou d'isopropyle, le stéarate d'hexadécyle, d'isopropyle, d'octyle ou d'isodécyle, l'oléate de décyle, le laurate d'héxyle, le dicaprylate de propylène glycol, les esters dérivés d'acide lanolique comme le lanolate d'isopropyle et le lanolate d'isocétyle, les isoparaffines, les acétylglycérides, les octanoates d'alcools et de polyalcools tels ceux de glycol et de glycérol, les ricinoléates d'alcools et de polyalcools, les triglycérides d'acides gras ; les huiles de silicone telles que les cyclométhicones, les polydiméthylsiloxanes volatiles et/ou non volatiles ou encore les phényldiméthylsiloxanes, leurs mélanges.

La composition peut, en outre, contenir un ou plusieurs autres ingrédients classiquement utilisés dans les compositions nettoyantes et/ou de soin. Ces ingrédients sont des filtres, des parfums, des conservateurs, des antioxydants, des agents régulateurs de pH, des séquestrants, des charges, des colorants, des actifs cosmétiques ou dermatologiques. Ces adjuvants sont utilisés dans les proportions habituelles des compositions nettoyantes, et par exemple de 0,01 à 10 % du poids total de la composition. Ces adjuvants doivent être de nature et utilisés en quantités telles qu'ils ne perturbent pas les propriétés recherchées pour la composition de l'invention.

Comme actifs utilisables dans l'invention, on peut citer des antibactériens comme l'octopirox et le triclosan, des agents kératolytiques comme l'acide salicylique, des huiles essentielles, des vitamines.

La composition selon l'invention peut être préparée par tout moyen connu de l'homme du métier, et en particulier par simple mélange des différents constituants et moulage dans un moule adéquat. Cependant, de façon avantageuse, elle est préparée par mélange suivi d'un malaxage puis d'une extrusion dans un extrudeur, de préférence un extrudeur bi-vis tel que ceux décrits dans les documents EP-A-605284 et FR-A-2,715,306, et dans lequel les deux vis tournent dans le même sens. On peut aussi utiliser le procédé décrit dans le document EP-A-651991.

Les différents constituants de la composition sont introduits, à l'entrée de l'extrudeur bi-vis, dans la zone d'alimentation à température ambiante, de préférence environ 20°C. De préférence, on introduit les constituants solides en tête de l'extrudeur puis les constituants liquides sont introduits latéralement. Le tout est malaxé dans diverses zones de l'extrudeur, maintenues à une température allant, préférentiellement, de 15 à 25°C ; la masse obtenue est transportée vers la sortie de l'extrudeur et extrudée au travers d'une filière. La vitesse de rotation des vis est de l'ordre de 400 à 1000 tours/minute, de préférence comprise entre 550 et 650 tours/minute.

La masse extrudée sort de la filière sous la forme de boudins de diamètre donné selon la filière utilisée, pouvant être ensuite découpés et mis en forme, notamment, sous forme de bâton ou de pain solide. D'autres formes peuvent bien entendu être réalisées en choisissant des filières appropriées et des dispositifs de mise en forme des produits finaux, adaptés à la forme recherchée.

Ainsi, l'invention a encore pour objet un procédé de fabrication d'une composition pulvérulente pour le nettoyage et/ou le soin de la peau, contenant un liant cosmétiquement acceptable, et une charge pulvérulente insoluble dans ce liant et en quantité suffisante pour structurer ladite composition et lui conférer un aspect de solide déformable dans lequel le liant est emprisonné, la charge pulvérulente comprenant des particules solides de polymère thermoplastique et au moins un tensioactif sous forme pulvérulente, la dite composition étant apte à mousser lors de son hydratation, consistant à introduire dans un mélangeur extrudeur, de préférence à froid, le liant puis la charge, puis à mettre en forme la composition issue du mélangeur extrudeur dans une filière de forme voulue.

La masse extrudée peut également être déshydratée et/ou broyée et/ou compactée. La déshydratation est avantageusement utilisée lorsque les ingrédients de la composition sont introduits sous forme de solution ou de dispersion en milieu aqueux.

Du fait que la totalité du procédé d'extrusion est réalisée à température ambiante, de l'ordre de 20-25°C, il est possible d'utiliser des ingrédients sensibles à la chaleur, du type vitamines ou huiles volatiles.

D'autre part, les ingrédients sensibles à la chaleur peuvent être introduits dans n'importe quelle zone de l'extrudeur (en tête, au milieu ou en final) puisqu'aucune détérioration due à la chaleur n'est à craindre. Ceci est en particulier avantageux pour l'introduction des agents structurant du type Expancel.

Il est également possible d'effectuer une partie de l'extrusion sous gaz inerte (azote par exemple).

Les exemples ci-après sont donnés à titre illustratif et non limitatif en vue de mieux faire ressortir les caractéristiques de l'invention. Les quantités y sont données en % en poids.

### Exemple 1 : Masque moussant pulvérulent pour peaux grasses à tendance acnéique

- Cocoyl iséthionate de sodium (Jordapon Cl vendu par la société Jordan) (tensioactif anionique sous forme pulvérulente) 42,5 %
- Lauryl éther sulfate de monoéthanolamine vendu à 28 % dans l'eau par la société Henkel (Sipon LEM 235) 51 %
- Conservateur qs
- Octopirox 0,1 %
- Parfum qs
- Acide salicylique 0,5 %
- Expancel 551 DE 20 5,5 %

Le mode opératoire consiste à introduire à froid les tensioactifs en tête d'extrudeur, puis à introduire latéralement dans les différents étages de l'extrudeur bi-vis les actifs, les conservateurs, le parfum et, au final, l'Expancel. Le mélange est extrudé à froid jusqu'à obtention d'une pâte homogène, qui est ensuite mise en forme dans une filière appropriée pour obtenir un boudin que l'on découpe en bâton.

Le masque obtenu est une pâte blanche, non collante, facilement modelable, facilement mouillable ou hydratable, d'application et d'élimination faciles, douce au toucher et ayant un bon pouvoir moussant.

Ce masque peut alors être hydraté, puis malaxé jusqu'à ce qu'il mousse notamment dans le creux de la main. La mousse obtenue est alors appliquée en couche mince sur la peau du visage. La quantité d'eau peut représenter de 6 à 10 fois en volume celle de l'échantillon prélevé pour le nettoyage.

Dès l'application de la mousse sur le visage, on obtient la libération des impuretés. Ensuite, les actifs anti-acné pénètrent dans la peau pour la nettoyer en profondeur et la désinfecter en vue d'empêcher la formation de bouton. L'élimination du masque se fait simplement avec de l'eau.

### Exemple 2 : Masque moussant pour tout type de peaux

- Cocoyl iséthionate de sodium (Jordapon Cl vendu par la société Jordan) (tensioactif anionique sous forme pulvérulente) 40 %
- Expancel 551 DE 20 6 %
- Lauryl éther sulfate de monoéthanolamine vendu à 28 % dans l'eau par la société Henkel (Sipon LEM 235) 54 %

Le mode opératoire est le même que pour l'exemple 1 et on obtient une pâte ayant des propriétés analogues.

### Exemple 3 : Masque moussant pour peaux sensibles

- Cocoyl iséthionate de sodium (Jordapon Cl vendu par la société Jordan) (tensioactif anionique sous forme pulvérulente) 35 %
- Lauryl éther sulfate de monoéthanolamine vendu à 28 % dans l'eau par la société Henkel (Sipon LEM 235) 60 %
- Huile d'amande douce 1 %
- Microsphères EL 23 5 %

Le mode opératoire est le même que pour l'exemple 1 et on obtient une pâte ayant des propriétés analogues.

## Revendications

1. Composition moussante pulvérulente à hydrater pour le nettoyage et/ou le soin de la peau, contenant un liant cosmétiquement acceptable et une charge pulvérulente insoluble dans ce liant et en quantité suffisante pour structurer ladite composition et lui conférer un aspect de solide déformable dans lequel le liant est emprisonné, la charge pulvérulente comprenant des particules solides de polymère thermoplastique et au moins un tensioactif sous forme pulvérulente, la dite composition étant apte à mousser lors de son hydratation.

2. Composition selon la revendication 1, caractérisée en ce que les particules de polymère ont une granulométrie de 1 µm à 300 µm.

3. Composition selon la revendication 1 ou 2, caractérisée en ce que les particules de polymère ont une granulométrie de 10 µm à 100 µm.

4. Composition selon l'une des revendications précédentes, caractérisée en ce que les particules de polymère ont une densité inférieure à 0,09.

5. Composition selon l'une des revendications précédentes, caractérisée en ce que les particules de polymère ont une densité inférieure à 0,04.

6. Composition selon l'une des revendications précédentes, caractérisée en ce que la charge est présente en une concentration au moins égale à la charge pigmentaire volumique critique.

7. Composition selon l'une des revendications précédentes, caractérisée en ce que les particules de polymère sont creuses.

8. Composition selon l'une des revendications précédentes, caractérisée en ce que les particules de polymère sont réalisées en un matériau choisi parmi les polymères et copolymères de chlorure de vinylidène, de chlorure de vinyle, d'acrylonitrile et/ou d'un monomère acrylique ou styrénique.

9. Composition selon l'une des revendications précédentes, caractérisée en ce que les particules de polymère sont des particules creuses de copolymère de chlorure de vinylidène, d'acrylonitrile et/ou d'un monomère acrylique ou styrénique.

10. Composition selon l'une des revendications précédentes, caractérisée en ce qu'elle contient, en outre, au moins un tensioactif liquide moussant et/ou nettoyant faisant partie du liant.

11. Composition selon l'une des revendications précédentes, caractérisée en ce que le tensioactif liquide est lauryl éther sulfate de monoéthanolamine.

12. Composition selon la revendication 10 ou 11, caractérisée en ce que le tensioactif liquide est présent en une quantité allant de 20 à 88 % du poids total de la composition.

13. Composition selon l'une des revendications précédentes, caractérisée en ce que le tensioactif sous forme pulvérulente est présent en une quantité allant de 10 à 80 % du poids total de la composition.

14. Composition selon l'une des revendications précédentes, caractérisée en ce que le tensioactif sous forme pulvérulente est présent en une quantité allant de 30 à 60 % du poids total de la composition.

15. Composition selon l'une des revendications précédentes, caractérisée en ce que le tensioactif sous forme pulvérulente est le cocoyl iséthionate de sodium.

16. Composition selon l'une des revendications 11 à 15, caractérisée en ce que le tensioactif liquide est présent à raison de 33 % à 67 % du poids total de la composition.

17. Composition selon l'une des revendications précédentes, caractérisée en ce qu'elle contient, en outre, au moins un ingrédient choisi parmi les filtres, les parfums, les conservateurs, les antioxydants, les agents régulateurs de pH, les séquestrants, les charges, les colorants, les actifs cosmétiques ou dermatologiques, leurs mélanges.

18. Composition selon l'une des revendications précédentes, caractérisée en ce qu'elle est obtenue par extrusion à froid.

19. Utilisation cosmétique de la composition selon l'une des revendications précédentes pour le nettoyage et/ou le soin de la peau d'êtres humains.

20. Procédé cosmétique de nettoyage et/ou de soin de la peau, consistant à hydrater une composition selon l'une des revendications 1 à 18, à la faire mousser, puis à l'appliquer sur la peau et enfin à rincer la peau.

21. Procédé de fabrication d'une composition pulvérulente pour le nettoyage et/ou le soin de la peau, contenant un liant cosmétiquement acceptable, et une charge pulvérulente insoluble dans ce liant et en quantité suffisante pour structurer ladite composition et lui conférer un aspect de solide déformable dans lequel le liant est emprisonné, la charge pulvérulente comprenant des particules solides de polymère thermoplastique et au moins un tensioactif sous forme pulvérulente, la dite composition étant apte à mousser lors de son hydratation, consistant à introduire dans un mélangeur extrudeur, le liant puis la charge, puis à mettre en forme la composition issue du mélangeur extrudeur dans une filière de forme voulue.

## Patentansprüche

1. Pulverförmige, zu hydratisierende, schäumende Zusammensetzung für die Reinigung und/oder die Pflege der Haut, die ein kosmetisch akzeptables Bindemittel und einen pulverförmigen Feststoff enthält, der in diesem Bindemittel unlöslich und in einer Menge enthalten ist, die ausreichend ist, um der Zusammensetzung Struktur zu geben und das Aussehen eines verformbaren Feststoffs zu verleihen, in dem das Bindemittel eingeschlossen ist, wobei der pulverförmige Füllstoff feste Partikel aus einem thermoplastischen Polymer und mindestens einen pulverförmigen grenzflächenaktiven Stoff enthält, wobei die Zusammensetzung bei ihrer Hydratisierung einen Schaum zu bilden vermag.

2. Zusammensetzung nach Anspruch 1, dadurch gekennzeichnet, daß die Polymerpartikel eine Partikelgröße von 1 bis 300 µm aufweisen.

3. Zusammensetzung nach Anspruch 1 oder 2, dadurch gekennzeichnet, daß die Polymerpartikel eine Partikelgröße von 10 bis 100 µm aufweisen.

4. Zusammensetzung nach einem der vorhergehenden Ansprüche, dadurch gekennzeichnet, daß die Polymerpartikel eine Dichte unter 0,09 aufweisen.

5. Zusammensetzung nach einem der vorhergehenden Ansprüche, dadurch gekennzeichnet, daß die Polymerpartikel eine Dichte unter 0,04 aufweisen.

6. Zusammensetzung nach einem der vorhergehenden Ansprüche, dadurch gekennzeichnet, daß der Füllstoff in einer Konzentration enthalten ist, die mindestens der kritischen, volumenbezogenen Pigmentkonzentration entspricht.

7. Zusammensetzung nach einem der vorhergehenden Ansprüche, dadurch gekennzeichnet, daß die Polymerpartikel Hohlpartikel sind.

8. Zusammensetzung nach einem der vorhergehenden Ansprüche, dadurch gekennzeichnet, daß die Polymerpartikel aus einem Material hergestellt sind, das unter den Polymeren und Copolymeren von Vinylidenchlorid, Vinylchlorid, Acrylnitril und/oder eines Acrylmonomers oder Styrolmonomers ausgewählt ist.

9. Zusammensetzung nach einem der vorhergehenden Ansprüche, dadurch gekennzeichnet, daß die Polymerpartikel Hohlpartikel aus einem Copolymer aus Vinylidenchlorid, Acrylnitril und/oder einem Acrylmonomer oder Styrolmonomer sind.

10. Zusammensetzung nach einem der vorhergehenden Ansprüche, dadurch gekennzeichnet, daß sie außerdem mindestens einen schaumbildenden und/oder reinigenden, flüssigen grenzflächenaktiven Stoff, der Teil des Bindemittels ist, enthält.

11. Zusammensetzung nach einem der vorhergehenden Ansprüche, dadurch gekennzeichnet, daß es sich bei dem flüssigen grenzflächenaktiven Stoff um das Monoethanolaminlaurylethersulfat handelt.

12. Zusammensetzung nach Anspruch 10 oder 11, dadurch gekennzeichnet, daß der flüssige grenzflächenaktive Stoff in einem Mengenanteil von 20 bis 88 % des Gesamtgewichts der Zusammensetzung enthalten ist.

13. Zusammensetzung nach einem der vorhergehenden Ansprüche, dadurch gekennzeichnet, daß der pulverförmige grenzflächenaktive Stoff in einem Mengenanteil von 10 bis 80 % des Gesamtgewichts der Zusammensetzung enthalten ist.

14. Zusammensetzung nach einem der vorhergehenden Ansprüche, dadurch gekennzeichnet, daß der pulverförmige grenzflächenaktive Stoff in einem Mengenanteil von 30 bis 60 % des Gesamtgewichts der Zusammensetzung enthalten ist.

15. Zusammensetzung nach einem der vorhergehenden Ansprüche, dadurch gekennzeichnet, daß es sich bei dem pulverförmigen grenzflächenaktiven Stoff um Natriumcocoylisethionat handelt.

16. Zusammensetzung nach einem der Ansprüche 11 bis 15, dadurch gekennzeichnet, daß der flüssige grenzflächenaktive Stoff in einem Anteil von 33 bis 67 % des Gesamtgewichts der Zusammensetzung enthalten ist.

17. Zusammensetzung nach einem der vorhergehenden Ansprüche, dadurch gekennzeichnet, daß sie außerdem mindestens einen Bestandteil enthält, der unter Filtern, Parfüms, Konservierungsmitteln, Antioxidantien, den pH-Wert regulierenden Mitteln, Maskierungsmitteln, Füllstoffen, Farbstoffen, kosmetischen oder dermatologischen Wirkstoffen, ihren Gemischen ausgewählt ist.

18. Zusammensetzung nach einem der vorhergehenden Ansprüche, dadurch gekennzeichnet, daß sie durch eine in der Kälte durchgeführte Extrusion hergestellt ist.

19. Kosmetische Verwendung der Zusammensetzung nach einem der vorhergehenden Ansprüche für die Reinigung und/oder die Pflege der Haut des Menschen.

20. Kosmetisches Verfahren zur Reinigung und/oder Pflege der Haut, das darin besteht, eine Zusammensetzung nach einem der Ansprüche 1 bis 18 zu hydratisieren, sie zum Schäumen zu bringen, sie dann auf die Haut aufzutragen und schließlich die Haut abzuspülen.

21. Verfahren zur Herstellung einer pulverförmigen Zusammensetzung für die Reinigung und/oder die Pflege der Haut, die ein kosmetisch akzeptables Bindemittel und einen pulverförmigen Füllstoff enthält, der in diesem Bindemittel unlöslich ist und in einer Menge vorhanden ist, die ausreichend ist, um der Zusammensetzung Struktur zu geben und das Aussehen eines verformbaren Feststoffs zu verleihen, in dem das Bindemittel eingeschlossen ist, wobei der pulverförmige Füllstoff feste Partikel aus einem thermoplastischen Polymer und mindestens einen pulverförmigen grenzflächenaktiven Stoff enthält, wobei die Zusammensetzung bei ihrer Hydratisierung einen Schaum zu bilden vermag, das darin besteht, in einen Extrudermischer das Bindemittel und dann den Füllstoff zu geben und anschließend die aus dem Extrudermischer austretende Zusammensetzung in einer Düse, die die gewünschte Form aufweist, zu formen.

## Claims

1. Foaming pulverulent composition to be hydrated for cleaning and/or caring for the skin which comprises a cosmetically acceptable binder and a pulverulent filler which is insoluble in this binder and which is present in an amount sufficient to structure the said composition and to confer on it the appearance of a deformable solid in which the binder is trapped, the pulverulent filler comprising solid particles of thermoplastic polymer and at least one surfactant in pulverulent form, the said composition being capable of foaming when it is hydrated.

2. Composition according to Claim 1, characterized in that the polymer particles have a particle size of 1 µm to 300 µm.

3. Composition according to Claim 1 or 2, characterized in that the polymer particles have a particle size of 10 µm to 100 µm.

4. Composition according to one of the preceding claims, characterized in that the polymer particles have a relative density of less than 0.09.

5. Composition according to one of the preceding claims, characterized in that the polymer particles have a relative density of less than 0.04.

6. Composition according to one of the preceding claims, characterized in that the filler is present at a concentration at least equal to the critical pigment volume concentration.

7. Composition according to one of the preceding claims, characterized in that the polymer particles are hollow.

8. Composition according to one of the preceding claims, characterized in that the polymer particles are prepared from a material chosen from polymers and copolymers of vinylidene chloride, of vinyl chloride, of acrylonitrile and/or of an acrylic or styrene monomer.

9. Composition according to one of the preceding claims, characterized in that the polymer particles are hollow particles of a copolymer of vinylidene chloride, of acrylonitrile and/or of an acrylic or styrene monomer.

10. Composition according to one of the preceding claims, characterized in that it additionally comprises at least one foaming and/or cleaning liquid surfactant forming part of the binder.

11. Composition according to one of the preceding claims, characterized in that the liquid surfactant is monoethanolamine lauryl ether sulphate.

12. Composition according to Claim 10 or 11, characterized in that the liquid surfactant is present in an amount ranging from 20 to 88% of the total weight of the composition.

13. Composition according to one of the preceding claims, characterized in that the surfactant in pulverulent form is present in an amount ranging from 10 to 80% of the total weight of the composition.

14. Composition according to one of the preceding claims, characterized in that the surfactant in pulverulent form is present in an amount ranging from 30 to 60% of the total weight of the composition.

15. Composition according to one of the preceding claims, characterized in that the surfactant in pulverulent form is sodium cocoyl isethionate.

16. Composition according to one of Claims 11 to 15, characterized in that the liquid surfactant is present in a proportion of 33% to 67% of the total weight of the composition.

17. Composition according to one of the preceding claims, characterized in that it additionally comprises at least one ingredient chosen from screening agents, fragrances, preservatives, antioxidants, pH-regulating agents, sequestering agents, fillers, dyes, cosmetic or dermatological active principles, or mixtures thereof.

18. Composition according to one of the preceding claims, characterized in that it is obtained by extrusion under cold conditions.

19. Cosmetic use of the composition according to one of the preceding claims for cleaning and/or caring for human skin.

20. Cosmetic process for cleaning and/or caring for the skin, which consists in hydrating a composition according to one of Claims 1 to 18, causing it to foam, then applying it to the skin and, finally, rinsing the skin.

21. Process for the manufacture of a pulverulent composition for cleaning and/or caring for the skin, which composition comprises a cosmetically acceptable binder and a pulverulent filler which is insoluble in this binder and which is present in an amount sufficient to structure the said composition and to confer on it the appearance of a deformable solid in which the binder is trapped, the pulverulent filler comprising solid particles of thermoplastic polymer and at least one surfactant in pulverulent form, the said composition being capable of foaming when it is hydrated, which process consists in introducing the binder and then the filler into a mixer-extruder and then shaping the composition emerging from the mixer-extruder in a die of desired shape.
